# EUROPEAN PATENT SPECIFICATION

(11) **EP 2 985 011 B1**
(45) Date of publication and mention of the grant of the patent: **07.10.2020**
(21) Application number: 15185023.7
(22) Date of filing: 15.11.2012
(51) Int. Cl.: A61F 5/44, A61M 1/00, A61B 5/20

(54) **FLUID COLLECTION AND EXPULSION APPARATUS**
FLÜSSIGKEITSSAMMEL UND AUSSTOSSVORRICHTUNG
APPAREIL DE COLLECTE ET D'EXPULSION DE FLUIDE

(30) Priority: 15.11.2011 GB 201119676
(43) Date of publication of application: 17.02.2016
(62) Divisional of application: 12794454.4
(73) Proprietor: Melio Medical Limited, Liverpool L3 9SJ (GB)
(72) Inventor: WILLS, Trevor, Chesterfield, Derbyshire S40 2JW (GB)
(74) Representative: Read, David Graham

(56) References cited:
- EP-A1- 0 610 638
- EP-A1- 2 145 611
- EP-A1- 2 243 448
- WO-A1-2009/142508

## Description

The present invention relates to fluid collection and expulsion apparatus and particularly, although not exclusively, where the fluid is one produced by a person, such as urine.

### BACKGROUND

Urinary incontinence can affect a wide range of people. In particular, many stroke victims suffer from urinary incontinence as a result of partial brain damage caused by their stroke. After the stroke, the individual may be otherwise healthy and urinary incontinence can severely restrict their lifestyle, not to mention be a cause of personal embarrassment.

Individuals suffering from urinary incontinence are generally prescribed leg bags. Leg bag systems typically comprise a urine bag that straps to the individual's leg and is connected by tubing to a catheter or other urine collection device worn by the individual. The urine bag has an outlet having a tap or valve for the individual or medical staff to empty the bag through.

In many cases, the outlet tap or valve is down by the individual's ankle making it difficult for some individuals to access if they are elderly or less physically able, for example. In these situations, another person such as a carer or family member must operate the tap or valve to empty the bag. This results in a loss of independence and possibly a loss of dignity for the individual.

As of the date of this application, three types of outlet tap or valve are in general usage on common leg bags. None of these are entirely leak proof and all suffer the problem of leakage at some stage during their use.

One particularly important consideration for a leg bag user is knowing when the bag requires emptying. If the bag becomes too full, urine can flow back up into the individual (known as "reflux") and present a serious infection risk to the individual. A common result of this problem is that leg bag users are reluctant to take in fluids, i.e. they drink less to avoid filling the bag frequently. This is not conducive to infection prevention. Furthermore, patients who are recovering from illness tend to suffer from dehydration, so reluctance to intake fluid will hinder their recovery.

A leg bag system that addresses some of these problems is described in the application WO-A-03/055423 (Wills, Trevor). The system comprises a leg bag having sensing means for detecting a fluid level in the bag and processing means for processing information received by the sensing means. The system further comprises signaling means for alerting the user when the fluid reaches a predetermined level, so that the user can empty the contents of the bag before it becomes too full. In some arrangements, the system further comprises a pump facilitating emptying of the bag via an upper outlet obviating the need for the individual to bend down and access a gravity-driven outlet tap or valve below the bag.

For hygiene reasons, leg bags need to be replaced at regular intervals. For example, to reduce the risk of infection but not prove too much of an inconvenience, typical leg bags may be replaced every seven days.

Leg bags utilizing electronic means such as sensors, controllers and pumps also require a power source to operate. Given that leg bags are designed to provide their users with more independence and freedom, a portable power source such as one or more batteries is appropriate. Rechargeable batteries are particularly suitable. For the convenience of the user, it is preferable that the batteries are able to power the electronic components for at least as long as a single bag is being used (e.g. seven days), before replacement or recharging of the batteries is required. Typically, however, it is found that leg bags using standard pumps, such as centrifugal pumps, draw too much current to be powered by a single battery power source over a seven day period. If smaller, less-current draining standard pumps are used, the required pumping pressure is not achieved to sufficiently eject fluid upward from the bag through an outlet.

Level detection may be done by electromechanical means, such as a floating component at the surface of the fluid which contacts an electrical contact at a set level within the bag. However, electromechanical measurement means are commonly large and cumbersome which is not desirable in a leg bag that seeks to be as small and discreet as possible. Additionally, the presence of moving parts has associated manufacturing and assembly costs and may also give rise to reliability issues in the device. Alternative leg bags use sensors to detect the fluid level by measuring a property of the urine, such as resistance, to ascertain the total volume. However, there is wide variation in the composition (e.g. material content and concentration) of urine produced by an individual which depends on a large number of variables, including the food and drink intake of the individual. Therefore, accurate determination of volume by measurement of urine properties becomes difficult, especially in very dilute urine. One solution to this is to use a "set-level", which covers a certain range of the parameter being measured, to indicate that a specified level (and hence volume) has been reached. The "set-level" might be changed to suit different individuals and their specific conditions. However, this is far from the ideal situation where accurate measurements can be taken from all individuals, regardless of their food and drink intake. In the absence of accurate measurements, false alarms may be activated when the level is below the level at which the bag requires emptying, possibly increasing the frequency that the user needs to empty the bag and thereby proving to be unnecessarily inconvenient.

A further consideration regarding the accuracy of measurement is the possibility of a moving fluid within the bag causing the level measurement means to falsely indicate that the bag requires emptying. This may arise as a result of the individual walking or otherwise moving with the leg bag attached to them. Clearly, false readings and subsequent false alarms would be inconvenient and irritating to the user.

WO-A-20009/142508 (Bonvik, Knut) describes a system for real time long-term recording and/or real time spectroscopy of a discharged urine amount from a patient. A registration unit is provided that includes one or more light sources and one or more sensor means for optical real time reading/registration of urine volume and/or real time spectroscopy of the urine volume.

EP-A-2243448 (Uni Charm Corp and Hitachi Ltd) describes an automatic urine disposal apparatus adapted to detect the presence of faeces in a wide range. A detector unit has a urination detector and a defecation detector. The urination detector comprises a pair of first electrodes and the defecation detector comprises a pair of second electrodes. The pair of first electrodes and the pair of second electrodes each have regions spaced one from another and extending in parallel one to another and these regions comprise portions that can be wetted with urine or moisture contained in faeces.

It is an object of the present invention to provide improved fluid collection and expulsion apparatus that improve on or overcome at least some of the problems associated with the prior art.

### BRIEF SUMMARY OF THE DISCLOSURE

The present invention is defined in claim 1 and concerns a portable fluid collection apparatus. In accordance with an example there is provided a centrifugal pump for use with a portable fluid collection apparatus for collecting fluid produced by a person, the centrifugal pump comprising:
a substantially cylindrical pump chamber having an inner diameter;
a fluid inlet in fluid communication with said pump chamber;
a fluid outlet in fluid communication with said pump chamber; and
an impeller having an outer diameter and being rotatably mounted on a driveshaft within said pump chamber intermediate said fluid inlet and said fluid outlet, where said driveshaft is rotatable by driving means to rotate said impeller in use and accelerate fluid flowing into said pump chamber through said fluid inlet and out of said fluid outlet;
wherein the inner diameter of the pump chamber is substantially equal to or greater than 1.40 times the outer diameter of the impeller.

In one preferable arrangement, the inner diameter of the pump chamber is equal to or less than 1.50 times the outer diameter of the impeller. In a further or alternative preferable arrangement, the inner diameter of the pump chamber is substantially equal to or greater than 1.42 times the outer diameter of the impeller. In a particularly preferable arrangement, the inner diameter of the pump chamber is between 1.42 and 1.45 times the outer diameter of the impeller, inclusive, or is between 1.42 and 1.43 times the outer diameter of the impeller, inclusive.

In any arrangement, the impeller preferably comprises a central spindle rotatably mounted on said driveshaft, and a plurality of circumferentially spaced blades extending radially from said central spindle, wherein said outer diameter of said impeller is the largest dimension of the impeller in a direction substantially perpendicular to said driveshaft.

A longitudinal axis of said fluid outlet is preferably arranged substantially perpendicularly relative to a longitudinal axis of said fluid inlet and radially aligned with said impeller.

In accordance with another example there is provided a portable fluid collection apparatus comprising:
a fluid reservoir for receiving fluid produced by a person, the fluid reservoir having a reservoir inlet and an reservoir outlet; and
a centrifugal pump according to the first aspect of the present invention, wherein the fluid inlet of the pump is in fluid communication with said reservoir outlet.

The portable fluid collection apparatus preferably further comprises an outlet conduit in fluid communication with the fluid outlet of said pump, and/or further preferably comprises a battery for powering said pump via driving means.

In one preferable arrangement said fluid reservoir is disposable and/or said pump is disposable.

The portable fluid collection apparatus preferably further comprises detection means for detecting a property of fluid within the fluid reservoir, wherein said detection means preferably comprise means for measuring a property of fluid between a first position and a second position spaced from said first position. Preferably, said detection means further comprises means for measuring a property of fluid between said first position and a third position, where the distance between said first position and said third position is less than the distance between said first position and said second position.

Said detection means preferably comprises means for measuring an electrical or optical property of fluid, and preferably comprises means for measuring one or more of electrical resistance, capacitance, electrical resonance and optical transmittance of fluid.

Preferably, said second position is located so as to come into contact with fluid when the fluid reservoir contains a volume of fluid that is between 55% and 75% of its maximum capacity, and preferably between 60% and 70% of its maximum capacity, and further preferably between 64% and 68% of its maximum capacity. Further preferably, said second position is located so as to come into contact with fluid when the fluid reservoir contains a volume of fluid that is about 66% of its maximum capacity.

The portable fluid collection preferably further comprises processor means configured to receive data from the detection means and determine the level of fluid within the fluid reservoir, and signal means for producing an audible, visual, or tactile signal, wherein said processor means is configured to activate said signal means to produce said signal when the determined level of fluid within the fluid reservoir exceeds a predetermined threshold.

Said processor means are preferably configured to calibrate measured data corresponding to a property of fluid between said first position and said second position using measured data corresponding to a property of fluid between said first position and said third position in order to determine the level of fluid within the fluid reservoir.

Preferably, said processor means are configured to receive a data packet from the detection means a plurality of times per second and is further configured to only activate said signal means to produce a signal when said processor means determines that the level of fluid within the fluid reservoir exceeds said predetermined threshold over a predetermined number of successive data packets.

In one preferable arrangement, said processor means are configured to receive a data packet from the detection means 15 times per second or more, and/or wherein said predetermined number of successive data packets is between 15 and 45, and preferably 30.

In another preferable arrangement, said processor means are configured to receive a data packet from the detection means 100 times per second, 125 time per second, 256 times per second, or more.

Said detection means preferably comprise one or more electrical wires for connecting said detection means to a power source and/or said processor means, and wherein said one or more wires pass out from the inside of said reservoir to the outside of said reservoir through an outlet fitting having a bore, wherein a seal around the one or more wires in the bore is formed by a heat shrinkable sleeve sheathing the one or more wires and a heat cured epoxy resin.

Alternatively, said detection means preferably comprise one or more electrical wires for connecting said detection means to a power source and/or said processor means, and wherein said one or more wires pass out from the inside of said reservoir to the outside of said reservoir through an outlet fitting having a bore, wherein a seal around the one or more wires in the bore is formed by an elastomeric sleeve sheathing the one or more wires.

In accordance with an aspect of the present invention there is provided a portable fluid collection apparatus comprising:
a fluid reservoir for receiving fluid produced by a person; and
detection means for detecting a property of fluid within the fluid reservoir;
wherein said detection means comprises means for measuring a property of fluid between a first position and a second position spaced from said first position and means for measuring a property of fluid between said first position and a third position, where the distance between said first position and said third position is less than the distance between said first position and said second position.

The portable fluid collection apparatus further comprises processor means configured to receive data from the detection means and determine the level of fluid within the fluid reservoir, said processor means being further configured to calibrate measured data corresponding to a property of fluid between said first position and said second position using measured data corresponding to a property of fluid between said first position and said third position in order to determine the level of fluid within the fluid reservoir.

Preferably, the portable fluid collection apparatus further comprises signal means for producing an audible, visual, or tactile signal, wherein said processor means is configured to activate said signal means to produce said signal when the determined level of fluid within the fluid reservoir exceeds a predetermined threshold.

Said detection means preferably comprises means for measuring an electrical or optical property of fluid and preferably comprises means for measuring one or more of electrical resistance, capacitance, electrical resonance and optical transmittance of fluid.

Said second position is preferably located so as to come into contact with fluid when the fluid reservoir contains a volume of fluid that is between 55% and 75% of its maximum capacity, and preferably between 60% and 70% of its maximum capacity, and further preferably between 64% and 68% of its maximum capacity.

Preferably, said second position is located so as to come into contact with fluid when the fluid reservoir contains a volume of fluid that is about 66% of its maximum capacity.

Said processor means are preferably configured to receive a data packet from the detection means a plurality of times per second and is further configured to only activate said signal means to produce a signal when said processor means determines that the level of fluid within the fluid reservoir exceeds said predetermined threshold over a predetermined number of successive data packets.

Preferably, said processor means are configured to receive a data packet from the detection means 15 times per second or more and/or wherein said predetermined number of successive data packets is between 15 and 45, and preferably 30.

Alternatively, said processor means are preferably configured to receive a data packet from the detection means 100 times per second, 125 times per second, 256 times per second, or more.

Said detection means preferably comprise one or more electrical wires for connecting said detection means to a power source and/or said processor means, and wherein said one or more wires pass out from the inside of said reservoir to the outside of said reservoir through an outlet fitting having a bore, wherein a seal around the one or more wires in the bore is formed by a heat shrinkable sleeve sheathing the one or more wires and a heat cured epoxy resin.

Alternatively, said detection means preferably comprise one or more electrical wires for connecting said detection means to a power source and/or said processor means, and wherein said one or more wires pass out from the inside of said reservoir to the outside of said reservoir through an outlet fitting having a bore, wherein a seal around the one or more wires in the bore is formed by an elastomeric sleeve sheathing the one or more wires.

In accordance with another example there is provided a portable fluid collection apparatus comprising:
a fluid reservoir for receiving fluid produced by a person;
detection means for detecting a property of fluid within the fluid reservoir;
processor means configured to receive data from the detection means and determine the level of fluid within the fluid reservoir; and
signal means for producing an audible, visual, or tactile signal, wherein said processor means is configured to activate said signal means to produce said signal when the determined level of fluid within the fluid reservoir exceeds a predetermined threshold;
wherein said detection means comprises means for measuring a property of fluid between a first position and a second position spaced from said first position;
said processor means being configured to receive a data packet from the detection means a plurality of times per second and being further configured to only activate said signal means to produce a signal when said processor means determines that the level of fluid within the fluid reservoir exceeds said predetermined threshold over a predetermined number of successive data packets.

Said processor means are preferably configured to receive a data packet from the detection means 15 times per second or more, and/or wherein said predetermined number of successive data packets is between 15 and 45, and preferably 30.

Alternatively, said processor means are preferably configured to receive a data packet from the detection means 100 times per second, 125 times per second, 256 times per second, or more.

Preferably, said detection means comprise one or more electrical wires for connecting said detection means to a power source and/or said processor means, and wherein said one or more wires pass out from the inside of said reservoir to the outside of said reservoir through an outlet fitting having a bore, wherein a seal around the one or more wires in the bore is formed by a heat shrinkable sleeve sheathing the one or more wires and a heat cured epoxy resin.

Alternatively, said detection means preferably comprise one or more electrical wires for connecting said detection means to a power source and/or said processor means, and wherein said one or more wires pass out from the inside of said reservoir to the outside of said reservoir through an outlet fitting having a bore, wherein a seal around the one or more wires in the bore is formed by an elastomeric sleeve sheathing the one or more wires.

### BRIEF DESCRIPTION OF THE DRAWINGS

Embodiments of the invention are further described hereinafter with reference to the accompanying drawings, in which:
Figure 1 is a perspective view of an impeller for use in a centrifugal pump in accordance with an example;
Figure 2 is a cross sectional view of a centrifugal pump in accordance with an example;
Figure 3 shows an outlet fitting according to an example;
Figure 4 shows front and rear sides of a detection means holder strip in accordance with an aspect of the present invention; and
Figure 5 shows an integrated pump, strip and outlet fitting in accordance with an aspect of the present invention.

### DETAILED DESCRIPTION

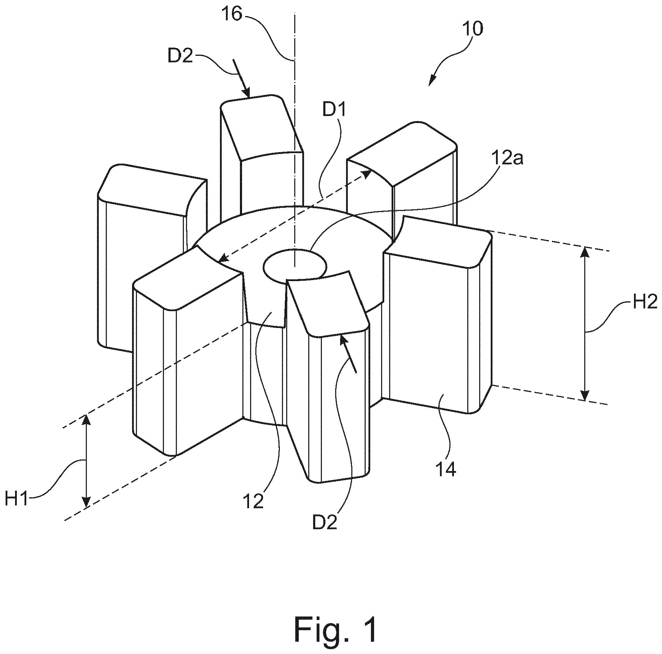

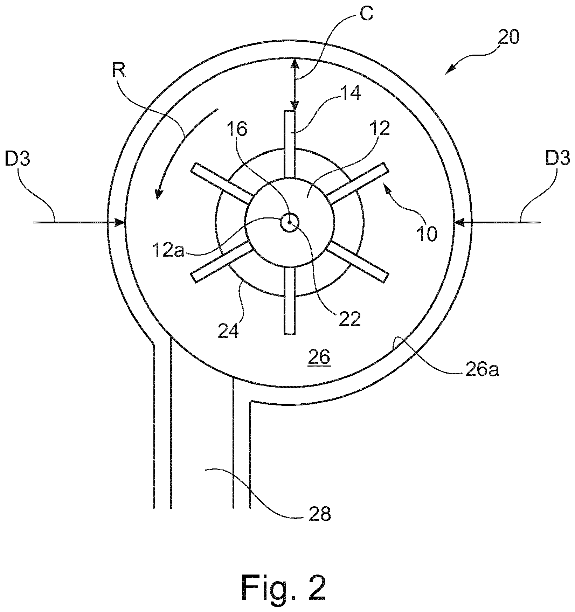

Figure 1 shows a detailed view of an impeller 10 of a centrifugal pump 20 which is shown in Figure 2. The impeller 10 comprises a central spindle 12 having a central hole 12a through which a drive shaft 22 (see Figure 2) may be disposed to rotatably mount the impeller 10 thereon. The impeller 10 has a central axis 16 which is parallel to the drive shaft 22 when rotatably mounted thereon. The central spindle 12 has a diameter D1 and an axial height H1.

The impeller 10 has a plurality of circumferentially spaced blades 14 extending radially from the central spindle 12. In the example shown in the Figures, the impellor has six blades 14 that each extend in a radial direction without any circumferential bend. However, in alternative arrangements, other configurations of blades are envisaged, such as blades that do extend radially with a circumferential bend (e.g. "spiral" blades). In all arrangements, however, the blades 14 define an outer diameter D2 of the impeller 10 and have an axial height dimension, H2. In the example shown in the Figures, the axial height H2 of the blades 14 is greater than the axial height H1 of the central spindle 12, although this need not necessarily be the case in other arrangements.

As shown in Figure 2, when assembled as part of the centrifugal pump 20, the impeller 10 is rotatably mounted on a driveshaft 22 within a pump chamber 26. The driveshaft 22 is connected to driving means (not shown), such as a motor, to rotate the driveshaft 22 and, in turn, rotate the impeller 10 in the direction indicated in Figure 2 by arrow R. The pump chamber 26 is generally cylindrical and is defined by a pump chamber wall 26a. The pump chamber wall 26a has an inner diameter D3 that is greater than the outer diameter D2 of the impeller 10 so as to completely envelope the impeller 10.

The pump chamber 26 is fluidly connected to a fluid inlet 24 and a fluid outlet 28, where the fluid inlet is centered on and substantially parallel to the central axis 16 of the impeller 10. The fluid outlet 28 is arranged perpendicularly relative to the fluid inlet 24 and is radially aligned with said impeller 10 such that fluid flowing into the pump chamber 26 through fluid inlet 24 is accelerated by rotating blades 14 of the impeller 10 and passes out of the fluid outlet 28. The rotating impeller 10 experiences fluidic drag as it rotates in the fluid within the pump chamber 26. A result of fluidic drag is that more current (i.e. more electrical power) is required to rotate the impeller 10 to produce a certain pumping pressure as compared to an ideal, drag free system that is otherwise identical. Thus, more electrical power is consumed than would be if fluidic drag was non-existent or of less effect.

In certain examples, the ratio of the outer diameter D2 of the impeller 10 relative to the inner diameter D3 of the pump chamber 26 is such that fluidic drag is reduced but the required pumping pressure can still be achieved (through acceleration of the fluid by the impeller 10). The fluidic drag is reduced such that the pump can be powered by a single portable power source, such as a battery pack, without the requirement of charging or replacing the power source for a desired period of time, such as seven days or more.

The pump 20 may be required to pump 500 ml of liquid vertically upward through a vertical tube of 1.5 m in 1 minute or less so that a user of a urinary leg bag can empty the contents of the bag without the need to bend down and open a tap or valve to allow gravity-driven expulsion of fluid. The pumping pressure for achieving these desirable conditions can be met using a power source, such as a battery, that does not require charging or replacing within a period of less than seven days.

To achieve the required pumping pressure but reduce fluidic drag to reduce the electric current required for operation, the inner diameter D3 of the pump chamber 26 may be substantially equal to or greater than 1.40 times the outer diameter D2 of the impeller 10. The inner diameter D3 of the pump chamber 26 is preferably between 12 and 16 mm, and is further preferably 14 mm. In a preferable arrangement, the inner diameter D3 of the pump chamber 26 is substantially equal to or less than 1.50 times the outer diameter D2 of the impeller 10. In a preferable arrangement, the inner diameter D3 of the pump chamber 26 is substantially equal to or greater than 1.42 times the outer diameter D2 of the impeller 10, and is preferably between 1.42 and 1.45 times the outer diameter D2 of the impeller 10, or further preferably between 1.42 and 1.43 times the outer diameter D2 of the impeller 10.

The inner diameter D3 of the pump chamber 26 may be 14 mm and the outer diameter D2 of the impeller 10 may be between 9.80 and 9.86 mm, inclusive. Further preferably, the central spindle 12 has a diameter D1 of about 4.89 mm and an axial height H1 of about 2.76 mm and/or the blades 14 have an axial height H2 of about 4.21 mm.

The pump 20 may have a clearance C such that fluidic drag is reduced thereby reducing the electrical demands of the pump 20, without impairing the pump's pumping ability below the threshold for correct operation as part of a urinary leg bag system. In a preferable embodiment, the fluid inlet 24 of the pump 20 is connected to a fluid reservoir (not shown) and the fluid outlet 28 is connected to an outlet conduit (not shown) which may be tubing which can be held, manipulated and directed by a user to control the expulsion of fluid whilst the pump is operating. As described above, in a urinary leg bag system, the outlet conduit may be about 1.5 m in length and may be oriented substantially vertically upwards relative to the pump 20, in use.

Where the centrifugal pump 20 forms part of a urinary leg bag system, the system may additionally comprise any features of known leg bag systems, such as the features described in WO-A-03/055423 (Wills, Trevor). For example, additional features may include fluid detection means to measure the level of fluid within the bag, processor means for processing data from the detection means, and signal means for alerting the user when the detected level of fluid reaches or exceeds a predetermined level.

The bag and/or pump 20 may be disposable such that it can be thrown away after a given time interval, such as seven days, to reduce infection risk. This supports a system where a battery can provide the required electrical power to the system over a seven day period. Variations of pump dimensions within the scope of the present invention permit pumps that can be supported over different time periods by a single portable power source to be realized. The battery may be conveniently recharged or replaced at the same time as changing the bag and/or pump 20. The pump 20 may be integral with the bag such that the two are disposable together. Preferably, the battery is rechargeable so as to reduce the disposal of hazardous materials commonly found in batteries. The pump 20 may be cleanable so that hygiene can be maintained without the need to regularly dispose of the pump 20.

In accordance with an aspect of the present invention, an improved fluid detection system for use on or in a fluid reservoir is provided. In a preferable embodiment, the fluid reservoir may form part of a portable fluid collection apparatus for receiving fluid produced by a person, such as a urinary leg bag system, although the improved detection system may be equally applicable to other fluid reservoirs. Detection means are provided for detecting a property of fluid within the fluid reservoir, where the detection means has means for measuring a property of fluid between a first position and a second position spaced from said first position. The detection means further has means for measuring a property of fluid between the first position and a third position. The distance between the first position and the third position is less than the distance between the first position and the second position.

Processor means are provided that are configured to receive data from the detection means and determine the level of fluid within the fluid reservoir. The processor means are further configured to calibrate measured data corresponding to a property of the fluid between the first position and the second position using measured data corresponding to a property of the fluid between the first position and the third position in order to determine the level of fluid within the fluid reservoir.

In a specific example, the first position is located at a lower part of the fluid reservoir, such as proximate to an outlet of the reservoir. The second position is chosen to be at a level that is equal to the level of fluid when the fluid reaches a threshold volume within the reservoir (i.e. a "threshold level"), above which reflux and infection become a significant possibility. In one example, a fluid reservoir filled with fluid to a volume of 66% of the maximum capacity of the reservoir is considered to be a suitable threshold level which should not be exceeded if reflux and infections are to be avoided.

The detection means measure a property of fluid between the first and second positions. This may be done using electrodes or other suitable measurement apparatus, such as emitters and receivers, located at each of the first and second positions. The measurement between the first and second positions should change depending on whether the first and second positions are connected to one another by fluid or not. That is, there should be a measurable difference between the first and second positions when the volume of fluid is such that it covers the first and second positions, and when it covers only one or neither of the positions. However, as discussed above, measureable properties of fluids, in particular urine, depend upon the given fluid's composition and concentration. In the case of urine, measurable electrical resistance will depend upon what the individual has had to eat or drink prior to passing the urine, among other variables. Therefore, the measurable difference between the case where urine does not link the first and second positions and the case where it does, may, in some cases, be slight, and possibly not defined enough to reliably and repeatedly determine that the urine has filled the reservoir enough to reach the second position. In an embodiment of the present invention, measurement between the first and third position is used to improve the accuracy of the determination of fluid level from data of measurements between the first and second positions, by calibration. In particular, the first and third positions are spaced closer to one another than the first and second positions. Therefore, the amount of fluid required to connect the first position to the third position will be less than the amount of fluid required to connect the first position to the second position. Over a smaller distance, measureable changes between a fluidly linked condition and a non-fluidly linked condition are more pronounced and reliable. One might assume then that the first and second positions should be located close to one another close to the desired threshold level (e.g. around 66% of maximum capacity), however use of close first and second positions may give rise to false readings if fluid within the reservoir was to slosh around and fluidly connect the positions despite the volume of fluid being below the desired threshold level. This is not a problem for the first and third positions if they are located such that it is highly likely that they will both be submerged in fluid, even at low volumes. Indeed, if the first and third positions are close to the bottom of the reservoir, gravity will ensure that even low volumes of fluid are able to fluidly connect the first and third positions so as to be measurable.

Upon measuring a property of the fluid between the first and third positions, the processor means can determine what sensitivity to operate at when attempting to establish whether measurements of the fluid between the first and second position are indicative of fluid connection or not (i.e. whether the volume of fluid within the reservoir is at the threshold level or not).

The detection means may measure an electrical or optical property of the fluid which may be one or more properties selected from the non-exhaustive list comprising electrical resistance, capacitance, electrical resonance and optical transmittance of the fluid.

In one example where electrical resistance is measured, first, second and third electrodes are located at the first, second and third positions, respectively, to take measurements therebetween. If a fluid has a high resistivity, the measured resistance will be less compared with a fluid having a lower resistivity. By first measuring the resistance between the first and third electrodes, the relative resistance of the fluid can be determined. If the fluid has a high resistivity, the change in resistance between the first and second electrodes will be small when the first and second electrodes change from being not fluidly connected to fluidly connected. However, the measurement between the first and third electrodes calibrates the measurement between the first and second electrodes such that the processor means can more accurately determine when the fluid connects the first and second electrodes (i.e. the fluid level reaches the second electrode). To put another way, the measurement between the first and third electrodes can determine the size of change required between the first and second electrodes for the processor means to determine that the fluid level has reached the second electrode (i.e. the predetermined threshold level has been met or exceeded). Of course, this is not exclusive to resistance measurements and electrodes. Other measurements of properties utilizing other detection means can make use of this arrangement, within the scope of the present invention.

As described above, the location of the second position determines what volume of fluid corresponds to the threshold level. This is preferably set so that reflux in a urinary leg bag system is unlikely or cannot occur, thereby minimizing the risk of infection to the user. In a preferable embodiment, the second position is located so as to come into contact with fluid when the fluid reservoir contains a volume of fluid that is between 55% and 75% of its maximum capacity, and preferably between 60% and 70% of its maximum capacity, and further preferably between 64% and 68% of its maximum capacity. In a particularly preferable embodiment, the second position is located so as to come into contact with fluid when the fluid reservoir contains a volume of fluid that is substantially equal to 66% of its maximum capacity.

In a particularly preferable embodiment where the fluid reservoir is part of a urinary bag system, signal means are provided for producing an audible, visual, or tactile signal, wherein the processor means are configured to activate the signal means to produce said signal when the determined level of fluid within the fluid reservoir exceeds a predetermined threshold.

In a further preferable embodiment, measurements between the first and third positions are used to indicate when the bag is empty or close to empty. This measurement may then be used to automatically switch off the pump (either immediately or after a set time period) when emptying the fluid reservoir. This arrangement will avoid unnecessary usage of the power source and pump and will help prolong the operable usage lifetime of the system.

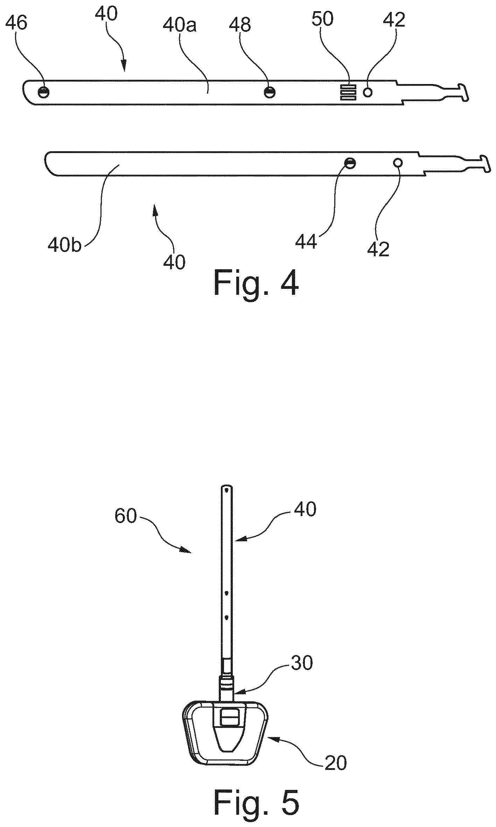

In accordance with a particularly preferable embodiment, a holder for the detection means is shown in Figure 4 as an elongate strip 40. In particular, a front side 40a of the strip 40 and a rear side 40b of the strip 40 are shown in Figure 4. The strip 40 has a lower aperture 42 to allow the passage of electrical wires therethrough. Additionally, the strip 40 has a first slotted peg on the rear side 40b, and a second slotted peg 46 and a third slotted peg 48 on the front side 40a. The first 44, second 46 and third slotted pegs 48 each allow an electrical wire to be secured thereabout so as to provide means to make measurements at each of the second and third positions, relative to the first position. A separating element 50 has channels for ensuring that electrical wires remain separate from one another along the strip 40, thereby avoiding short circuits. In use, the strip 40 is disposed in the fluid reservoir so that the second slotted peg 46 (which corresponds to the second position) is at the predetermined fluid level threshold. In alternative embodiments, the slotted pegs 44, 46, 48 may all be on the same side of the strip 40 or may be in alternative arrangements.

The strip 40 may take on other shapes to that shown in Figure 4 and may be used to hold other detection means, which may include sensors, probes, wires or other measurement apparatus.

In a further embodiment of the present invention, measurements are taken between the first and second positions a plurality of times per second to produce a data packet for each measurement that is sent to the processor means. The processor means then activates the signal means to produce a signal when the processor means determines that the level of fluid within the fluid reservoir exceeds the predetermined threshold level over a predetermined number of successive data packets, or a predetermined number of times within a particular time interval.

This arrangement may be used when there are only first and second positions for measurement or first, second and third measurement positions. It may also, although not necessarily, be combined with the above-described centrifugal pump 20.

The purpose of this arrangement is to reduce the likelihood of false readings that may arise because of turbulence in the fluid within the fluid reservoir. Using many measurements increases the likelihood that a positive result indicates that the fluid level has reached the second position, i.e. the predetermined threshold level.

In a particularly preferable embodiment, the detection means operate in one of two states, which may be named "WET" and "DRY", for example. If the processor means determines that the level of fluid within the fluid reservoir exceeds the predetermined threshold level for each of a predetermined number of successive data packets, then the detection means are set to the WET state. If the processor means determines that the level of fluid within the fluid reservoir is below the predetermined threshold level for each of a predetermined number of successive data packets, then the detection means are set to the DRY state. After being set to the WET state following the predetermined number of successive data packets, the processor means then activates the signal means to produce a signal to indicate that the fluid level has reached the predetermined level. Fewer than the predetermined number of successive data packets will not result in a switching of the state of the detection means.

In the case where electrical resistance is being used to determine the fluid level, a resistance above a predetermined resistance threshold indicates that the fluid within the fluid reservoir is below a predetermined threshold level, and a resistance above a predetermined resistance threshold indicates that the fluid within the fluid reservoir is at or above a predetermined threshold level.

In one embodiment, the processor means are configured to receive a data packet from the detection means 15 times per second or more, 50 times per second or more, or 125 times per second or more. In an alternative embodiment, the processor means are configured to receive a data packet from the detection means 256 times per second or more. In one example of a preferred embodiment, the predetermined number of successive data packets required to switch the detection means between WET and DRY states is 30, where preferably the processor means are configured to receive a data packet from the detection means 15 times per second. In this specific example, consistent readings would be required over a minimum period of 2 seconds for the detection means to switch between WET and DRY states. This would ensure, or at least increase the likelihood that the results are genuinely representative of the actual volume of fluid within the reservoir.

Furthermore, in embodiments where measurements between a first position and a third position are used to calibrate measurements between the first position and a second position, as described above, following the detection means being switched to the WET state following the processor means determining that the level of fluid within the fluid reservoir exceeds the predetermined threshold level for each of predetermined number of successive data packets, a predetermined measurement threshold (such as a resistance threshold) may be set using measurement between the first position and third positions to determine what value of measurement between the first position and second position is indicative of the predetermined threshold level of fluid within the fluid reservoir. The predetermined measurement threshold may be set each time the detection means is switched to the WET state following the processor means determining that the level of fluid within the fluid reservoir exceeds the predetermined threshold level for each of the predetermined number of successive data packets.

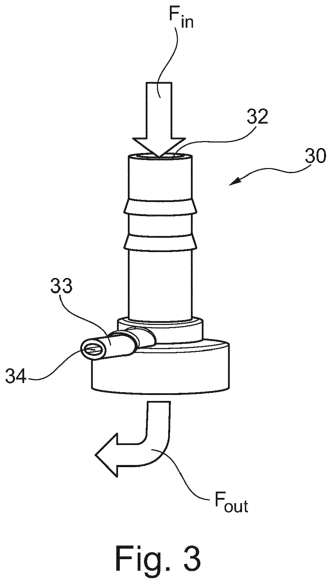

In any embodiment where electrical wires form part of the detection means, there exists a challenge to assemble the system such that the wires can extend from the inside of the fluid reservoir where they may be connected to electrodes or other measuring apparatus, to the outside of the reservoir where they may be connected to the processor means and/or a power supply, without creating a path along which fluid can flow and undesirably exit the fluid reservoir. In a urinary leg bag system, the available space is too small to use a moulded plug with connector pins. One solution in accordance with the present invention is to use an outlet fitting 30 such as the one shown in Figure 3. The outlet fitting 30 is attachable to an outlet of the fluid reservoir and has a central bore 32 through which fluid can flow in to the fitting (indicated by arrow Fin) and out (indicated by arrow Fout). The outlet fitting 30 additionally includes a branch 33 having a bore 34 in fluid communication with the central bore 32. Wires of the detection means may pass out of the inside of the reservoir into the fitting 30 through central bore 32 and out of the bore 34 of branch 33. The wires can then be sealed around the bore 34 of the branch 33 so that fluid can not exit the central bore 32 via the bore 34 of the branch 33. In one embodiment, sealing is achieved using a small sleeve of heat shrinkable tubing that is partially filled with an epoxy resin that cures rapidly when heat is applied during shrinking of the sleeve. In another embodiment, sealing is achieved by using wires that are sheathed in an elastomeric (e.g. rubber) tubing. The wires may be pre-moulded in the sheath, for example. The sheathed wires can then be inserted through the bore 34 of the branch 33 allowing the elastomeric nature of the sheath to provide a seal so that fluid cannot escape through the bore 34 of the branch 33.

In a preferable embodiment, the pump 20 and strip 40 (complete with detection means) and outlet fitting 30 are provided in one assembled unit 60, as shown in Figure 5. In use, the assembled unit 60 can be inserted into an opening, such as the fluid outlet, of the fluid reservoir. The unit 60 may be treated as disposable or may be cleaned at regular intervals to maintain cleanliness.

Any of the above-described features can be used in any suitable combination with any of the other above-described features, and the present invention is not necessarily limited to the specifically described combinations.

Throughout the description and claims of this specification, the words "comprise" and "contain" and variations of them mean "including but not limited to", and they are not intended to (and do not) exclude other moieties, additives, components, integers or steps. Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics, compounds, chemical moieties or groups described in conjunction with a particular aspect, embodiment or example of the invention are to be understood to be applicable to any other aspect, embodiment or example described herein unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The invention is defined by the claims and is not restricted to the details of any foregoing embodiments.

## Claims

1. A portable fluid collection apparatus comprising:
a fluid reservoir for receiving fluid produced by a person;
detection means (40) for detecting a property of fluid within the fluid reservoir;
and processor means configured to receive data from the detection means (40) and determine the level of fluid within the fluid reservoir;
wherein said detection means (40) comprises means for measuring a property of fluid between a first position (44) and a second position (46) spaced a first distance from said first position (44) **characterised in that** said detection means (40) further comprises means for measuring a property of fluid between said first position (44) and a third position (48), where a second distance between said first position (44) and said third position (48) is less than the first distance between said first position (44) and said second position (46);
said processor means being further configured to calibrate measured data corresponding to a property of fluid between said first position (44) and said second position (46) using measured data corresponding to a property of fluid between said first position (44) and said third position (48) in order to determine the level of fluid within the fluid reservoir.

2. A portable fluid collection apparatus according to claim 1, further comprising signal means for producing an audible, visual, or tactile signal, wherein said processor means is configured to activate said signal means to produce said signal when the determined level of fluid within the fluid reservoir exceeds a predetermined threshold.

3. A portable fluid collection apparatus according to claim 1 or 2, wherein said detection means (40) comprises means for measuring an electrical or optical property of fluid.

4. A portable fluid collection apparatus according to any preceding claim, wherein said detection means (40) comprises means for measuring one or more of electrical resistance, capacitance, electrical resonance and optical transmittance of fluid.

5. A portable fluid collection apparatus according to any preceding claim, wherein said second position (46) is located so as to come into contact with fluid when the fluid reservoir contains a volume of fluid that is between 55% and 75% of its maximum capacity, and preferably between 60% and 70% of its maximum capacity, and further preferably between 64% and 68% of its maximum capacity.

6. A portable fluid collection apparatus according to claim 5, wherein said second position (46) is located so as to come into contact with fluid when the fluid reservoir contains a volume of fluid that is about 66% of its maximum capacity.

7. A portable fluid collection apparatus according to any preceding claim, wherein said processor means are configured to receive a data packet from the detection means (40) a plurality of times per second and is further configured to only activate said signal means to produce a signal when said processor means determines that the level of fluid within the fluid reservoir exceeds said predetermined threshold over a predetermined number of successive data packets.

8. A portable fluid collection apparatus according to claim 7, wherein said processor means are configured to receive a data packet from the detection means 15 times per second or more and/or wherein said predetermined number of successive data packets is between 15 and 45, and preferably 30.

9. A portable fluid collection apparatus according to claim 7, wherein said processor means are configured to receive a data packet from the detection means (40) 100 times per second, 125 times per second, 256 times per second, or more.

10. A portable fluid collection apparatus according to any preceding claim, wherein said detection means (40) comprise one or more electrical wires for connecting said detection means (40) to a power source and/or said processor means, and wherein said one or more wires pass out from the inside of said reservoir to the outside of said reservoir through an outlet fitting having a bore, wherein a seal around the one or more wires in the bore is formed by a heat shrinkable sleeve sheathing the one or more wires and a heat cured epoxy resin.

11. A portable fluid collection apparatus according to any preceding claim, wherein said detection means (40) comprise one or more electrical wires for connecting said detection means (40) to a power source and/or said processor means, and wherein said one or more wires pass out from the inside of said reservoir to the outside of said reservoir through an outlet fitting having a bore, wherein a seal around the one or more wires in the bore is formed by an elastomeric sleeve sheathing the one or more wires.

## Patentansprüche

1. Tragbare Flüssigkeitssammelvorrichtung, umfassend:
ein Flüssigkeitsreservoir zum Empfangen einer von einer Person erzeugten Flüssigkeit;
Erfassungsmittel (40) zum Erfassen einer Eigenschaft der Flüssigkeit im Flüssigkeitsreservoir; und Prozessormittel, welche konfiguriert sind, um Daten von den Erfassungsmitteln (40) zu empfangen und den Füllstand der Flüssigkeit im Flüssigkeitsreservoir zu bestimmen; wobei die Erfassungsmittel (40) Mittel zum Messen einer Eigenschaft der Flüssigkeit zwischen einer ersten Position (44) und einer zweiten Position (46) umfassen, welche von der ersten Position (44) in einem ersten Abstand angeordnet ist; **dadurch gekennzeichnet, dass** die Erfassungsmittel (40) ferner Mittel zum Messen einer Eigenschaft der Flüssigkeit zwischen der ersten Position (44) und einer dritten Position (48) umfassen, wobei der zweite Abstand zwischen der ersten Position (44) und der dritten Position (48) kleiner als der Abstand zwischen der ersten Position (44) und der zweiten Position (46) ist;
wobei die Prozessormittel ferner konfiguriert sind, um die gemessenen Daten zu kalibrieren, welche einer Eigenschaft der Flüssigkeit zwischen der ersten Position (44) und der zweiten Position (46) entsprechen, durch Verwendung der gemessenen Daten, welche einer Eigenschaft der Flüssigkeit zwischen der ersten Position (44) und der dritten Position (48) entsprechen, um den Füllstand der Flüssigkeit im Flüssigkeitsreservoir zu bestimmen.

2. Tragbare Flüssigkeitssammelvorrichtung nach Anspruch 1, ferner umfassend Signalmittel zum Erzeugen eines hörbaren, sichtbaren oder taktilen Signals, wobei die Prozessormittel konfiguriert sind, um die Signalmittel zu aktivieren, um das Signal zu erzeugen, wenn der bestimmte Füllstand der Flüssigkeit im Flüssigkeitsreservoir eine vorbestimmte Schwelle überschreitet.

3. Tragbare Flüssigkeitssammelvorrichtung nach Anspruch 1 oder 2, wobei die Erfassungsmittel (40) Mittel umfassen, um eine elektrische oder optische Eigenschaft der Flüssigkeit zu messen.

4. Tragbare Flüssigkeitssammelvorrichtung nach einem der vorhergehenden Ansprüche, wobei die Erfassungsmittel (40) Mittel zum Messen eines oder mehrerer von einem elektrischen Widerstand, einer Kapazität, einer elektrischen Resonanz und einem optischen Transmissionsgrad der Flüssigkeit umfassen.

5. Tragbare Flüssigkeitssammelvorrichtung nach einem der vorhergehenden Ansprüche, wobei die zweite Position (46) so angeordnet ist, dass sie mit der Flüssigkeit in Berührung kommt, wenn das Flüssigkeitsreservoir ein Flüssigkeitsvolumen zwischen 55% und 75% seiner maximalen Kapazität und bevorzugt zwischen 60% und 70% seiner maximalen Kapazität, und weiter bevorzugt zwischen 64% und 68% seiner maximalen Kapazität enthält.

6. Tragbare Flüssigkeitssammelvorrichtung nach Anspruch 5, wobei die zweite Position (46) so angeordnet ist, dass sie mit der Flüssigkeit in Berührung kommt, wenn das Flüssigkeitsreservoir ein Flüssigkeitsvolumen von zirka 66% seiner maximalen Kapazität enthält.

7. Tragbare Flüssigkeitssammelvorrichtung nach einem der vorhergehenden Ansprüche, wobei die Prozessormittel konfiguriert sind, um ein Datenpaket von den Erfassungsmitteln (40) mehrmals pro Sekunde zu empfangen, und ferner konfiguriert sind, um die Signalmittel zur Erzeugung eines Signals nur dann zu aktivieren, wenn die Prozessormittel bestimmen dass der Füllstand der Flüssigkeit im Flüssigkeitsreservoir die vorbestimmte Schwelle über eine vorbestimmte Anzahl aufeinanderfolgender Datenpakete überschreitet.

8. Tragbare Flüssigkeitssammelvorrichtung nach Anspruch 7, wobei die Prozessormittel konfiguriert sind, um ein Datenpaket von den Erfassungsmitteln 15 Mal pro Sekunde oder mehr zu empfangen und/oder wobei die vorbestimmte Anzahl von aufeinanderfolgenden Datenpaketen zwischen 15 und 45 liegt und bevorzugt 30 beträgt.

9. Tragbare Flüssigkeitssammelvorrichtung nach Anspruch 7, wobei die Prozessormittel konfiguriert sind, um ein Datenpaket von den Erfassungsmitteln (40) 100 Mal pro Sekunde, 125 Mal pro Sekunde, 256 Mal pro Sekunde oder mehr zu empfangen.

10. Tragbare Flüssigkeitssammelvorrichtung nach einem der vorhergehenden Ansprüche, wobei die Erfassungsmittel (40) einen oder mehrere elektrische Drähte umfassen, um die Erfassungsmittel (40) mit einer Stromquelle und/oder mit den Prozessormitteln zu verbinden, und wobei der eine oder die mehreren Drähte aus dem Inneren des Reservoirs zur Außenseite des Reservoirs durch ein Ausgangselement durchdringen, welches eine Bohrung aufweist, wobei eine Dichtung um den einen oder die mehreren Drähten in der Bohrung durch eine wärmeschrumpfbare Hülse, welche den einen oder die mehreren Drähte umhüllt, und ein wärmehärtendes Epoxydharz gebildet ist.

11. Tragbare Flüssigkeitssammelvorrichtung nach einem der vorhergehenden Ansprüche, wobei die Erfassungsmittel (40) einen oder mehrere elektrische Drähte umfassen, um die Erfassungsmittel (40) mit einer Stromquelle und/oder den Prozessormitteln zu verbinden, und wobei der eine oder die mehreren Drähte aus dem Inneren des Reservoirs zur Außenseite des Reservoirs durch ein Ausgangselement durchdringen, welches eine Bohrung aufweist, wobei eine Dichtung um den einen oder die mehreren Drähte in der Bohrung durch eine elastomere Hülle gebildet ist, welche den einen oder die mehreren Drähte umhüllt.

## Revendications

1. Appareil de collecte de fluide portatif comprenant :
un réservoir de fluide permettant de recevoir un fluide produit par une personne ;
un moyen de détection (40) permettant de détecter une propriété de fluide à l'intérieur du réservoir de fluide ;
et un moyen de traitement conçu pour recevoir des données à partir du moyen de détection (40) et pour déterminer le niveau de fluide à l'intérieur du réservoir de fluide ;
ledit moyen de détection (40) comprenant un moyen de mesure d'une propriété de fluide entre une première position (44) et une deuxième position (46) espacée d'une première distance de ladite première position (44) ;
**caractérisé en ce que** ledit moyen de détection (40) comprend en outre un moyen de mesure d'une propriété de fluide entre ladite première position (44) et une troisième position (48), une seconde distance entre ladite première position (44) et ladite troisième position (48) étant inférieure à la première distance entre ladite première position (44) et ladite deuxième position (46) ;
ledit moyen de traitement étant en outre conçu pour étalonner les données mesurées correspondant à une propriété de fluide entre ladite première position (44) et ladite deuxième position (46) en utilisant des données mesurées correspondant à une propriété de fluide entre ladite première position (44) et ladite troisième position (48) afin de déterminer le niveau de fluide à l'intérieur du réservoir de fluide.

2. Appareil de collecte de fluide portatif selon la revendication 1, comprenant en outre un moyen de signalisation permettant de produire un signal audible, visuel ou tactile, ledit moyen de traitement étant conçu pour activer ledit moyen de signalisation afin de produire ledit signal lorsque le niveau de fluide déterminé à l'intérieur du réservoir de fluide dépasse un seuil prédéfini.

3. Appareil de collecte de fluide portatif selon la revendication 1 ou 2, ledit moyen de détection (40) comprenant un moyen de mesure d'une propriété électrique ou optique de fluide.

4. Appareil de collecte de fluide portatif selon une quelconque revendication précédente, ledit moyen de détection (40) comprenant un moyen de mesure de résistance électrique, de capacité, de résonance électrique et/ou de transmittance optique de fluide.

5. Appareil de collecte de fluide portatif selon une quelconque revendication précédente, ladite deuxième position (46) étant située de façon à venir en contact avec le fluide lorsque le réservoir de fluide continent un certain volume de fluide qui est compris entre 55 % et 75 % de sa capacité maximale, et de préférence entre 60 % et 70 % de sa capacité maximale, et encore mieux entre 64 % et 68 % de sa capacité maximale.

6. Appareil de collecte de fluide portatif selon la revendication 5, ladite deuxième position (46) étant située de façon à venir en contact avec le fluide lorsque le réservoir de fluide contient un certain volume de fluide qui est d'environ 66 % de sa capacité maximale.

7. Appareil de collecte de fluide portatif selon une quelconque revendication précédente, ledit moyen de traitement étant conçu pour recevoir un paquet de données à partir du moyen de détection (40) une pluralité de fois par seconde et étant en outre conçu pour uniquement activer ledit moyen de signalisation afin de produire un signal lorsque ledit moyen de traitement détermine que le niveau de fluide à l'intérieur du réservoir de fluide dépasse ledit seuil prédéfini sur un nombre prédéfini de paquets de données successifs.

8. Appareil de collecte de fluide portatif selon la revendication 7, ledit moyen de traitement étant conçu pour recevoir un paquet de données à partir du moyen de détection 15 fois par seconde ou davantage et/ou ledit nombre prédéfini de paquets de données successifs étant compris entre 15 et 45, et valant de préférence 30.

9. Appareil de collecte de fluide portatif selon la revendication 7, ledit moyen de traitement étant conçu pour recevoir un paquet de données à partir du moyen de détection (40) 100 fois par seconde, 125 fois par seconde, 256 fois par seconde ou plus.

10. Appareil de collecte de fluide portatif selon une quelconque revendication précédente, ledit moyen de détection (40) comprenant un ou plusieurs fils électriques permettant de connecter ledit moyen de détection (40) à une source d'alimentation et/ou audit moyen de traitement, et les un ou plusieurs fils sortant de l'intérieur dudit réservoir vers l'extérieur dudit réservoir à travers un raccord de sortie ayant un trou, un joint situé autour du ou des fils présent(s) dans le trou étant formé par un manchon thermorétractable enveloppant le ou les fils et par une résine époxy thermodurcie.

11. Appareil de collecte de fluide portatif selon une quelconque revendication précédente, ledit moyen de détection (40) comprenant un ou plusieurs fils électriques permettant de connecter ledit moyen de détection (40) à une source d'alimentation et/ou audit moyen de traitement, et le ou les fils électriques sortant de l'intérieur dudit réservoir vers l'extérieur dudit réservoir, à travers un raccord de sortie doté d'un trou, un joint situé autour du ou des fils présent(s) dans le trou étant formé par un manchon élastomère enveloppant le ou les fils.
